Europäisches Patentamt

(19) European Patent Office

Office européen des brevets

(11) **EP 0 749 697 A1**

## (12) EUROPEAN PATENT APPLICATION

(43) Date of publication:
27.12.1996 Bulletin 1996/52

(51) Int. Cl.$^6$: **A23P 1/08**, A23L 1/09,
A23L 1/29, A23L 1/10

(21) Application number: 95109753.4

(22) Date of filing: 22.06.1995

(84) Designated Contracting States:
BE CH DE DK ES FR GB IT LI NL SE

(71) Applicant: HERCULES INCORPORATED
Wilmington, Delaware 19894-0001 (US)

(72) Inventors:
• Lapré, John Arthur
NL-6715 HB Ede (NL)
• McNabola, William Thomas
NL-1271 DA Huizen (NL)

• Veenstra, Jan
NL-3771 KS Barneveld (NL)
• De Vries, Hielke Tjeerd
NL-6713 BV Ede (NL)

(74) Representative: Hansen, Bernd, Dr. Dipl.-Chem.
et al
Hoffmann, Eitle & Partner,
Patentanwälte,
Arabellastrasse 4
81925 München (DE)

### (54) Coated food

(57) Provided is the use of a cation cross-linked polysaccharide coating for reducing the glycemic response of a carbohydrate-containing food. The cation cross-linked polysaccharide coating may be formed from calcium cross-linked pectin or pectinate. The carbohydrate-containing food may be a rice grain, pasta shape, flour, starch or a piece of breakfast cereal.

Such a coated food may be prepared by coating a cross-linkable polysaccharide on a hydratable carbohydrate-containing food core. The food core is then hydrated by cooking the coated food core in an aqueous solution of cross-linking cations which simultaneously cross-link the coating. The resulting foods can be used in the diets of individuals suffering from diabetes, hypoglycemia or glycogen storage disease. They also have the effect of suppressing appetite or assisting in the performance of sustained physical activity.

The effect of coating the carbohydrate-containing food is slow down its rate of digestion in the small intestine. This in turn smooths out the release of glucose into the blood stream which otherwise would peak very sharply soon after the digestion of the food commences.

## Description

The present invention relates to a coated food, and in particular to a coated carbohydrate-containing food in which the coating has the effect of reducing an individual's glycemic response to the food. The invention also relates to a method for preparing the coated food, and its use as a food having modified digestive characteristics compared to an otherwise identical food without coating.

In the human digestive system, food is mechanically ground up and chemically broken down into small molecules that can be used by the body. Digestive enzymes from salivary, gastric, pancreatic, and intestinal glands hydrolyze proteins to amino acids, starch to simple sugars, and fats to glycerol and fatty acids. The digestive system consists essentially of the mouth, oesophagus, stomach, small intestine and large intestine. The stomach acts as a reservoir which releases food slowly into the small intestine. Digestion is completed in the small intestine where the food is absorbed into the bloodstream or lymph vessels. The small intestine is around 5 meters in length and it normally takes food around 4-5 hours to pass through it before reaching the large intestine.

In the human diet, carbohydrates carry energy and supply carbon atoms to biosynthetic pathways. They are found in nature as structural elements in most types of cells and tissues. Based on the number of monomeric units which they contain, carbohydrates are classified as monosaccharides, disaccharides, oligosaccharides (chains of 3-10 monosaccharide units), or polysaccharides. Starch is one of the most common naturally occuring polysaccharides. The monomeric unit of starch is glucose. Other common polysaccharides are glycogen and cellulose. These are respectively the carbohydrate used to store energy by animals and the substance forming the skeleton of plant structures. The monomeric unit of both of these polysaccharides is also glucose.

Starch is present within plant cells as discrete microscopic granules enclosed in phospholipid membranes. The size and form of a granule is often characteristic of the plant of its origin. Within each granule, amylopectin forms a branched, helical, crystalline system with the linear amylose dispersed within the amylopectin structure. The digestability of raw starch depends upon the accessibility and crystalline structure of its granules. The granules are gelatinised during cooking and freshly cooked starchy foods are easily digestible.

In western countries, starch constitutes about 60% of carbohydrates consumed. Starch is hydrolyzed in the intestinal tract into oligo-, di- and mono- saccharides. The enzymatic break-down of polysaccharides, such as starch, into oligosaccharides actually starts in the saliva. However, the bulk of starch breakdown occurs in the small intestine. Here, enzymatic splitting takes place of poly- and oligo- saccharides into low-molecular, and hence absorbable, monosaccharides such as glucose. Starch break-down in the small intestine can be a quite rapid process so that the majority of the starch is broken down within one hour of entering the small intestine from the stomach.

The total starch (TS) content of a food is measured as the yield of glucose from a finely milled or homogenized sample of the food in which the starch is completely gelatinised at 100°C, treated with potassium hydroxide to ensure complete dispersion of the starches to an amorphous, digestible form and then enzymatically digested with pancreatin and amylogluocosidase.

Starches can be classified into a number of different types or fractions according to their behaviour when incubated with enzymes without prior exposure to dispersing agents.

Three types of starch can be identified according to how they react to controlled periods of enzymatic digestion of homogenized and non-homogenized food samples. Firstly, rapidly digestible starch (RDS) consists mainly of amorphous and dispersed starch. It is typically found in high amounts in starchy foods which have been cooked by moist heat such as bread and potatoes. It is measured chemically as the starch which is converted into the constituent glucose molecules within 20 minutes of enzyme digestion. The next type is slowly digestible starch (SDS), which like RDS is in normal circumstances completely digested in the small intestine. However, its digestion proceeds more slowly than RDS. This fraction includes for example physically inaccessible amorphous starch. It is measured chemically as the starch converted to glucose after a further 100 minutes of enzyme digestion. The final starch type is resistant starch (RS) which may potentially resist digestion in the small intestine. It is measured chemically as the difference between TS obtained from the homogenized and chemically treated sample and the sum of RDS and SDS generated from non-homogenized food samples by enzyme digestion.

RS can be further sub-divided into three sub-types. These are $RS_1$ which is starch resistant to digestion because it is in a physically inaccessible form such as partly milled grains and seeds; $RS_2$ which has a granular form which is particularly resistant to enzyme digestion; and $RS_3$ which is the starch fraction most resistant to digestion and which mainly consists of retrograded amylose formed during the cooling of gelatinised starch.

RS which is not digested in the small intestine passes through to the large intestine where it is fermented by colonic bacteria to produce short chain fatty acids, carbon dioxide and methane. It is evident that the speed at which a starchy food is digested will depend upon the relative proportion which it contains of RDS, SDS and the different RS sub-types.

The amounts and relative amounts of RDS, SDS and RS in foods are highly variable. They depend partly on the source of starch, but also on the type and extent of processing which the food is subjected to. It should be noted that, for most starchy foods, cooking converts starch to a readily digestible form. The Table below provides data for the *in vitro* digestibility of starch in a variety of foods. The values are obtained by carrying out assays in accordance with the techniques described above.

| In vitro digestibility of starch in a variety of foods | | | | | |
|---|---|---|---|---|---|
| | % RDS | % SDS | RS$_1$ | RS$_2$ | RS$_3$ |
| Flour, white | 38 | 59 | - | 3 | t |
| Shortbread | 56 | 43 | - | - | t |
| Bread, white | 94 | 4 | - | - | 2 |
| Bread, wholemeal | 90 | 8 | - | - | 2 |
| Spaghetti, white | 55 | 36 | 8 | - | 1 |
| Biscuits, made with 50% raw banana flour | 34 | 27 | - | 38 | t |
| Biscuits, made with 50% raw potato flour | 36 | 29 | - | 35 | t |
| Peas, chick, canned | 56 | 24 | 5 | - | 14 |
| Beans, dried, freshly cooked | 37 | 45 | 11 | t | 6 |
| Bean, red kidney, canned | 60 | 25 | - | - | 15 |

In the above Table, values are expressed as a percentage of the total starch present in the food. The index "t" represents that only a trace of the particular starch type could be detected. It is evident from the data set out in the above Table that different foods have quite different relative contents of the different starch types.

When starch is digested in the small intestine it causes the level of glucose present in the blood to rise. The level of blood glucose in a healthy individual usually lies in the range 75-125 mg/100 ml. A greater glucose level can lead to heart, circulation, eye and kidney problems. A lower glucose level can lead to fatigue, fainting and hypoglycemic shock.

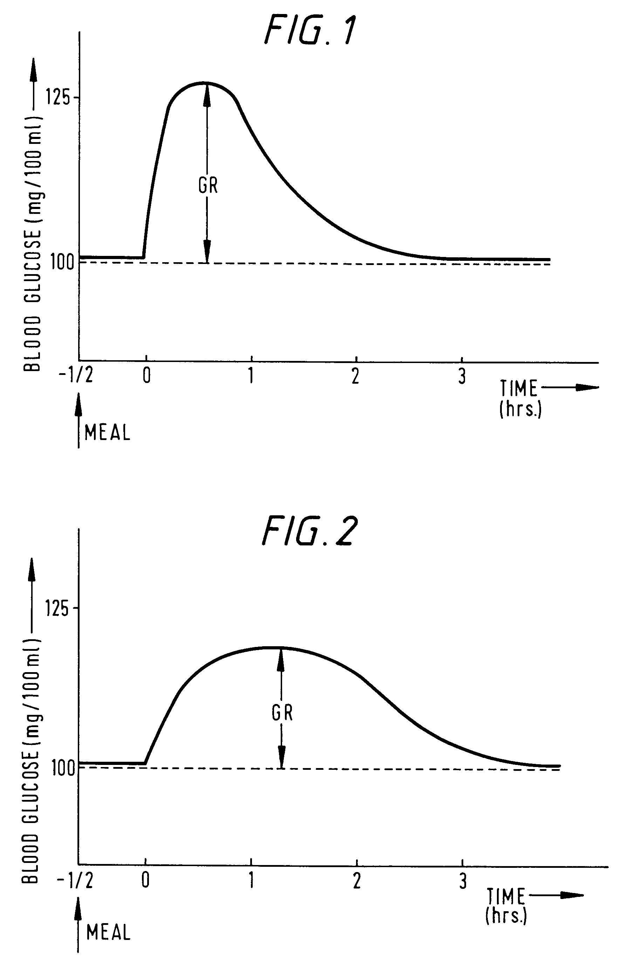

When a carbohydrate-containing meal is consumed, there is a characteristic rise and then fall in the blood glucose level over a period of about 3 hours. This is illustrated in Figure 1. The blood glucose level does not rise immediately because the food must first pass through the stomach before entering the small intestine. This takes around 0.5-1 hours. Once the food enters the small intestine from the stomach, then the blood glucose rises from its fasting level of about 100 mg/100 ml. This is caused by the digestion of polysaccharides such as starch into glucose, and then the entry of this from the gut and liver into the peripheral blood circulation. The subsequent fall to fasting levels is due to the action of insulin, secreted by the pancreas in response to various meal-related stimuli including glucose. Insulin causes the blood glucose level to fall by increasing the membrane transport, utilization, and storage of glucose from the blood in insulin-sensitive cells. Insulin acts at specialised transporter sites that exist for glucose within the plasma membrane of many cell types, notably in muscle and fat cells.

The height of the curve in Figure 1, labelled GR, is defined in this specification to be the "glycemic response" of the food. The precise glycemic response of a food will vary according to the amount of food eaten and from individual to individual depending upon such factors as the efficiency of their digestive system, and whether they suffer from diabetes. However, the absolute value of the glycemic response of a food is not important in the context of the present invention. Instead, what is important is how the glycemic response of the food varies between its normal (uncoated) form and a coated form as provided by the present invention. There are however a number of basic principles concerning the glycemic response of a food. For instance, food which includes a substantial content of RDS will possess a higher glycemic response, weight for weight, than a food formed substantially from SDS. In turn, such SDS-containing foods will give rise to a greater glycemic response than foods formed mostly from RS. In general, slowly digested foods produce flatter glycemic responses. Such foods have been termed "lente carbohydrate foods".

Several other characteristics of a food can affect its glycemic response. These include its particle size and texture, and any disruption which has taken place of its cell wall structure. Thus, cooked rice grains give rise to a smaller glycemic response than cooked flour. Cracking or milling of cereal grains to produce progressively greater disruption and finer particle sizes result in increased glycemic response values. Foods of coarse consistency possess smaller glycemic response values when swallowed without chewing, reinforcing the importance of particle size.

As mentioned above, it is important for an individual to control his or her blood glucose within a certain range. The two factors of greatest importance to enable this control are the activity of the liver and the balance of hormones. The liver has a certain autonomy. It is presently understood to have sensors which monitor the blood glucose level. When the level is high, the liver removes glucose from the blood; when the level is low, it releases glucose into the blood.

In the hormonal regulation of blood glucose, the balance between insulin and glucagon is of prime importance.

These two hormones are secreted by the pancreas in varying ratios, depending primarily upon the prevailing concentration of blood glucose. Any increase in the blood glucose level stimulates increased secretion of insulin, whereas a decrease in the blood glucose level stimulates the secretion of glucagon.

Some individuals are unable to control their blood glucose level by their own naturally produced insulin. This leads to the disease of diabetes. This disease is characterised by an above normal concentration of glucose in the blood. Diabetes is presently the third leading cause of death in the United States, where it kills 300,000 people per year. In 1950, there were 1.2 million diabetics in the US, in 1975 there were 5 million diabetics and in 1991 there were more than 11 million diabetics. Accordingly, it is increasingly important to provide effective palliatives for this disease.

There are two types of diabetes which have different underlying causes. In Type 1 or insulin-dependent diabetes, there is an absolute deficiency of insulin and the patient may require regular injections of insulin to maintain glycemic control. In addition to insulin, diet and exercise must be carefully regulated in order to maintain good blood-sugar control.

In contrast, in Type 2 or non-insulin dependent diabetes, the pancreas is producing insulin, although it may not be doing so at normal levels. Although insulin is present, blood glucose levels are still abnormal because the body does not respond to it. However, the cause of this insulin resistance is presently unknown. Of people who are diagnosed as having this form of diabetes, 80% are over-weight. Some non-insulin-dependent diabetics can control their condition through diet and exercise alone. Other diabetics may need a combination of diet, exercise and medications. Medications for this type of diabetes include a class of drugs called oral hypoglycemic agents that help non-insulin-dependent diabetics use blood glucose better.

A related disease, hypoglycemia, is caused by excessive circulating insulin. This is normally a result of an accidental overdose of insulin by a diabetic. This excess insulin results in a lowering of the blood glucose level. This particularly affects the brain which utilizes glucose as its main source of energy. Mild hypoglycemia can result in a lack of coordination. If the insulin excess is great enough, convulsions may occur, followed by coma.

Carbohydrates are an important part of a diabetic's diet. It is evident that diabetics should usually avoid foods having a high glycemic response i.e., those which result in a relatively high level of blood glucose soon after digestion. Instead, diabetics require foods having a relatively low glycemic response which produce a slower rate of glucose release into the blood. Slowing the rate of release of glucose into the blood reduces the risk of both hyperglycemia and hypoglycemia.

A slow release of glucose into the blood can be achieved by reducing the rate of digestion of the food. Thus, if the speed of digestion can be lowered, the initial rush of glucose from RDS can be prevented and instead replaced by a controlled and lengthier digestive process along the whole length of the small intestine leading to slow release of glucose into the blood stream. Foods which only slowly release glucose into the blood stream may also be advantageous in suppressing the appetite of an individual, and for assisting an individual to perform sustained physical activity. In the former case, the presence of an above normal level of glucose in an individual's bloodstream may cause a feeling of satiety and so discourages snacking. In the latter case, the continual release of glucose into the bloodstream assists an individual to perform strenuous exercise as the glucose acts as a readily accessible form of fuel to working muscles.

US-A-2517595 and US-A-2611708 disclose encasing food products in a film of pectinate or pectate. Such films are indicated to be tender and edible and can be eaten with the food itself. The films dissolve in boiling water. It is presumed that this is because the coated foods are uncooked, and swell when placed in boiling water due to their hydration causing the coating layer to be cast off and apparently dissolve.

US-A-5360614 discloses a food core formed from a metabolizable carbohydrate coated with a time delay release layer. This layer retards the release of carbohydrate into the digestive system and so reduces the glycemic response of the food. The coating layer is formed from stearic acid, hydrogenated or partially hydrogenated oils, such as cotton seed, soybean or rapeseed oil, calcium stearate or stearyl alcohol. The coating layer is formed by dissolving its components in an organic solvent such as ethanol in a coating pan, and then adding the food to be coated.

The coated foods of US-A-5360614 have a number of disadvantages. Firstly, many of the proposed coating agents are fatty substances which many people wish to avoid in their diet. Secondly, such coating agents are coated using a non-aqueous organic solvent and so are expensive and not appropriate in the home. It would be preferred if an aqueous coating method could be developed. The coatings based on fatty substances are not heat stable and disintegrate on heating above around 60°C. Therefore food coated according to this reference could not be cooked in boiling water without the fatty coating being lost. Finally, the coating method results in relatively large amounts of the coating material being deposited on the food. For instance, the food product of Example 6 of this reference is formed from 100 g of corn starch (the food), and 140 g of hydrogenated tallow and ethyl cellulose (the coating). Accordingly, the final coated food is around 58% by weight coating.

In the specification which follows, the term "food" is defined to be any material which is edible or can be rendered edible by any form of processing such as cooking. The term "food" therefore encompasses food ingredients such as a flour granule or granules, a starch granule or granules and raw vegetables.

On the other hand, the term "food product" used in the following specification refers to a food in an edible form ready for eating such as cooked rice.

One object of the present invention is to provide the use of a coating for reducing the glycemic response of a carbohydrate-containing food.

A further object is to provide a coated food comprising a core of a carbohydrate-containing hydrated food and a coating layer which results in the food having a lower glycemic response than the food without coating.

A further object is to provide a food which is suitable for cooking which comprises a core of a carbohydrate-containing food and components for forming a coating on its surface during cooking.

According to a further object, the invention seeks to provide a method for preparing a coated carbohydrate-containing food by coating a first component of a coating system on a hydratable carbohydrate-containing food core and then hydrating the food core by cooking the coated food core in an aqueous solution including a second component of the coating system which reacts with the first component to produce the final coat.

A further object of the invention is to provide coated carbohydrate-containing alimentary materials as a food for an individual suffering from diabetes, hypoglycemia or glycogen storage disease. According to a final object, the use is provided of a coated carbohydrate-containing food as an appetite suppressant or to assist an individual to perform sustained physical activity.

According to one aspect, there is provided the use of a cation cross-linked polysaccharide coating for reducing the glycemic response of a carbohydrate-containing food.

Such a use can have non-therapeutic effects such as suppressing appetite, and assisting an individual to perform sustained physical activity. Such effects are achieved because the presence of the coating reduces the rate of digestion of the carbohydrate in the small intestine. In normal-type foods, there is a relatively rapid release of glucose into the bloodstream following digestion of all of the RDS and some of the SDS. In contrast, coating the food with the cation cross-linked polysaccharide coating according to the present invention substantially prevents any digestion occurring in the stomach and then slows down the rate of digestion of the food as it passes through the small intestine so that the rate at which glucose is released into the bloodstream is reduced. This in turn results in the coated food giving rise to a lower glycemic response.

The above effect of the present invention is illustrated in Figure 2. In this Figure the same carbohydrate-containing meal is eaten at time (t) =-½ as in Figure 1 except that the food has been coated with a cation cross-linked polysaccharide. This coating prevents the initial surge in blood glucose found for uncoated food. Instead it retards the food's digestion resulting in glucose being released into the bloodstream at a slower rate and over a longer period of time. Thus, the areas under the curves in Figures 1 and 2 will be substantially equal. The cation cross-linked polysaccharide coating provided around a food according to the present invention can be viewed as converting the RDS and SDS of the food to a $RS_1$ type. It will be recalled that $RS_1$ is resistant to digestion because it is in a physically inaccessible form. In effect the function of the polysaccharide coating is to prevent the digestive system of the small intestine from rapidly digesting SDS and RDS present in the carbohydrate-containing food because these are rendered inaccessible by the coating.

In a further aspect of the invention, the use is provided of a cation cross-linked polysaccharide in the coating of a carbohydrate-containing food for reducing the glycemic response of the food. Such a use can have all of the non-therapeutical effects discussed above, and also a therapeutic effect as a food for an individual who suffers from diabetes, hypoglycemia or glycogen storage disease. Such coated foods are useful for people suffering from these diseases because they do not cause an initial surge in the level of blood glucose after being eaten but instead give rise to a slower rate of glucose release spread over an extended period of time.

The polysaccharide which is used as a component of the coating in the present invention is preferably alginate, alginic acid, pectin, pectinic acid, a pectinate, a pectate, polygalacturonic acid, carrageenan, cross-linkable cellulose or a derivative thereof, xanthan gum, agar, cross-linkable starch or a cross-linkable guar gum. It is particularly preferred that the polysaccharide is alginate, alginic acid, pectin, pectinic acid, a pectinate or a pectate.

The above polysaccharide may be cross-linked with any suitable cations. These include calcium cations and/or magnesium cations.

The coating should preferably extend over at least 50% of the surface area of the food. It is apparent that in areas where the food is uncoated, then digestion in the small intestine of this part of the food can proceed at the normal pace. In this case, the effect of coating the remaining surface area of the food is diminished. Accordingly, it is most preferred that the cross-linked polysaccharide coating should surround the food so as to fully encase it.

The polysaccharide coating around the food is relatively thin so that the weight of the coating makes up only a small fraction of the coated food product. The coated food comprises around 0.01-5% by weight (on a dry weight basis) of the cation cross-linked polysaccharide. More preferably, the coated food comprises 0.1-4%, and most preferably 1-3% by weight (on a dry weight basis) of the cation cross-linked polysaccharide.

The carbohydrate contained in the food may include any one or more of a peptidoglycan, polysaccharide, oligosaccharide, disaccharide, monosaccharide or sugar alcohol. In particular, the carbohydrate may be one or more of starch, dextrin, sucrose, mannose, maltose, glucose, fructose, lactitol, xylitol, sorbitol, lactose and mannitol. Most preferably, the carbohydrate includes at least starch.

The food itself may be any carbohydrate-containing food. Such foods include for example rice grains, pasta shapes such as tubes, spirals and shells, or a vegetable or part thereof. Such parts of a vegetable include cubes, dies, slices or chips. The food may also be a piece of breakfast cereal such a corn flake, puffed rice or shredded wheat. The food may also be a food ingredient for combining with other ingredients such as a flour granule or granules or a starch granule or granules. Such food ingredients may be included in puddings, candy bars, food bars or in instant foods such as soups or dessert mixes.

It will be appreciated that if the piece of food which is coated is relatively large, then it will be chewed in the mouth. This will reduce the effectiveness of the coating because parts of the coating may be stripped away and uncoated parts from the interior of the food will become exposed by the chewing action. This tendency of a food to be chewed is reduced if the food is relatively moist on its surface, e.g. boiled vegetable cubes or slices. This is because such moist food is relatively easy to swallow and requires little chewing. The susceptibility of a food to be chewed also of course depends on its size. The smaller the pieces of food are, the lower the tendency is to chew them. It is therefore preferred that the food has a longest dimension of less than 3 cm, and preferably less than 1 cm. Such foods include grains of rice which tend not to be chewed to any significant extent so that very little of the interior of the rice grains becomes exposed during chewing. The food on the other hand may have an elongate shape but with a relatively small cross-section. Such foods have relatively little of their interior exposed when chewed. It is therefore also preferred, as an alternative, that the food has an elongate shape with a cross-sectional area of no more than 1 $cm^2$, preferably less than 0.1 $cm^2$. Such elongate foods include spaghetti and french fries.

As mentioned above, the coated food may be already in an edible form. Alternatively, the food may be a food ingredient such as a flour granule or granules or a starch granule or granules. Such food ingredients can then be used in combination with other food ingredients to prepare a food product. In this case, the food product may comprise at least 10%, preferably at least 25%, more preferably at least 50% and most preferably at least 75% by weight of one or more coated food ingredients.

Englyst et al. describe methods of measuring starch fractions in their paper "Classification and measurement of nutritionally important starch fractions" published in the European Journal of Clinical Nutrition (1992), 46 (Suppl. 2), pages 33-50. In particular, Englyst et al. describe how free glucose (FG) may be measured in a sample which has been subjected to an *in vitro* incubation which attempts to mimic natural digestion. When subjecting a coated food according to the present invention to the *in vitro* Englyst assay method, it is preferred that the glycemic response of the coated food is reduced by at least 20% compared to an otherwise identical sample of an uncoated food 30 minutes after the start of the *in vitro* digestion. Thus, the amount of FG liberated from the coated food should be at least 20% less than that liberated from the uncoated food following 30 minutes of *in vitro* digestion.

It is further preferred that the glycemic response of the coated food should also be reduced by at least 20% compared to the uncoated food 60 minutes after *in vitro* digestion. More preferably, the glycemic response after both the 30 minute and 60 minute assays should be at least 30% less compared to the uncoated foods, and most preferably at least 40% less.

Using the same *in vitro* Englyst assay method, it is preferred that after one hour, no more than 80% of the polysaccharide present in the food should be released. More preferably, no more than 60% and most preferably no more than 50% of the polysaccharide should be released. The percentage of the carbohydrate which is released varies according to the thickness of the coating. Thus, the thicker the coating of the cation cross-linked polysaccharide, then the slower the rate of glucose release from the food into the assay system.

According to a further aspect, the present invention provides a coated food comprising a core of a carbohydrate-containing hydrated food and a coating layer comprising a cation cross-linked polysaccharide which is insoluble in boiling water. In such an aspect, the hydrated food may be for example a cooked grain of rice or pasta shape. Such a coated food can be prepared for example by boiling rice in an aqueous solution including 2% by weight of pectin or alginate for around 10-20 minutes. The boiled rice is then dried to form so-called parboiled rice. This in turn is then cooked in an aqueous solution including about 0.5-3% by weight of any suitable calcium salt such as calcium chloride for 2-10 minutes. The resulting food comprises a core of a hydrated rice grain and a coating layer comprising calcium cross-linked pectin or alginin. Because the cation cross-linked polysaccharide coating is formed whilst the rice is cooking, the rice grains have an opportunity to hydrate and swell before the cross-linked coating becomes fully formed. Whilst cross-linking of the polysaccharide is taking place, the polysaccharide is somewhat elastic and so can stretch to some extent as the rice grains swell due to their hydration. Accordingly, the cation cross-linked polysaccharide coating surrounding the rice grain is not removed by boiling water.

Such a method should be contrasted with forming a cation cross-linked polysaccharide around an uncooked rice grain. On cooking in boiling water, such a rice grain swells as it becomes hydrated causing the coating layer to peel away and so apparently to be dissolved as described in US-A-2517595 and US-A-2611708.

According to a further aspect, a food is provided for cooking which comprises:

(i) a core of a carbohydrate-containing food coated with a cross-linkable polysaccharide coating; and
(ii) a source of cross-linking cations for cross-linking the polysaccharide,

the components (i) and (ii) being separate or in physical admixture.

In such a food, the amount of cross-linking cations should preferably be 0.01-20% by weight based on the amount of the cross-linkable polysaccharide initially coated on the carbohydrate-containing food. Such a food may be a grain of parboiled rice. The source of cross-linking cations may be any suitable edible salt which can act as a suitable source of cross-linking cations such as calcium chloride, calcium sulphate, magnesium chloride or magnesium sulphate.

According to this aspect, the food is marketed in a form which can be prepared for eating following a one-step cooking procedure. Thus, the components (i) and (ii) are merely mixed together in water and cooked by for example boiling or simmering. This mixing of the components causes the cross-linking cations to cross-link with the cross-linkable polysaccharide thus forming a cation cross-linked polysaccharide coating around the food cores. As previously described, because the polysaccharide coating does not become fully cross-linked until after the food cores have been hydrated, the resulting cation cross-linked polysaccharide coating is resistant to boiling water. Thus, whilst the food is hydrating in the water, the cross-linkable polysaccharide surrounding the food is sufficiently elastic that it does not peel away as the food swells due to hydration.

The above cooking method forms a further aspect of the invention which is to prepare a cation cross-linked polysaccharide coated carbohydrate-containing food. This method comprises the steps of:

(i) coating a cross-linkable polysaccharide on a hydratable carbohydrate-containing food core; and
(ii) hydrating the food core by cooking the coated food core in an aqueous solution of cross-linking cations which simultaneously cross-link the coating.

The cross-linkable polysaccharide can be coated on the food core by spraying or dipping the food core with or in an aqueous solution of the polysaccharide at a temperature of 40-100°C. It is preferred that the aqueous solution should include 0.01-10%, more preferably 0.1-5%, by weight of the cross-linkable polysaccharide. Following the coating, the hydratable core is preferably dried prior to the hydrating step of cooking the food core in the presence of the cross-linking cations. In the case when the food core is a rice grain, the result of drying the rice grain coated with the cross-linkable polysaccharide is to produce so-called parboiled rice.

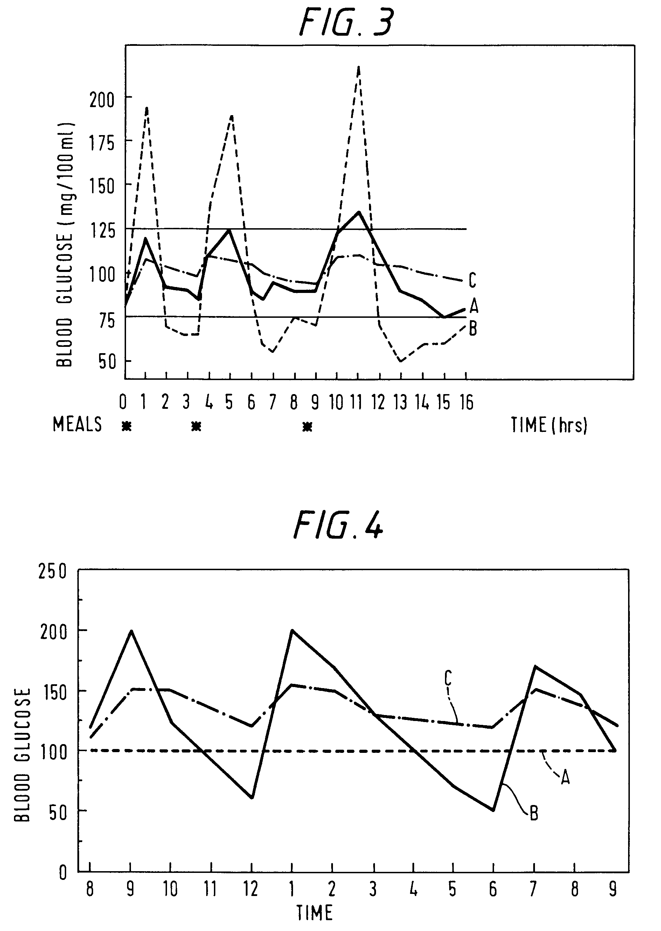

As mentioned above, the invention provides the use of a cation cross-linked polysaccharide coated carbohydrate-containing alimentary material as a food for an individual suffering from diabetes, hypoglycemia or glycogen storage disease. In the case of diabetes, the effect of such a use is illustrated in the graph of Figure 3. The line "A" represents the blood glucose level of a healthy person fed conventional (uncoated) carbohydrate-containing foods at times of 0, 3.5 and 8.5 hours. It will be seen that the blood glucose of the individual generally lies within the preferred range of 75-125 mg/100 ml. After each meal, the blood glucose rises as the carbohydrate content of the food is digested, and then subsides as insulin is released. The line "B" represents the blood glucose level of a diabetic fed with the same foods. It will be seen that the blood glucose level is poorly controlled, and rapidly rises after each meal because the individual is not able to control this using insulin. After all of the glucose has been released from the food after the meal, the blood glucose level falls as glucose is taken out of the blood stream by natural metabolism or for excretion in urine.

Finally, the line "C" represents the blood glucose level of a diabetic fed with the same foods as used in "A" and "B" above but which have been coated with a cation cross-linked polysaccharide. Such coated foods do not give rise to significant glucose peaks after each meal because the carbohydrate contained in the food is digested relatively slowly in the small intestine resulting in a slower release of glucose into the bloodstream. This slow release of glucose does not require moderating with insulin and so helps to control the blood glucose level of the diabetic.

The benefits of the coated foods for individuals who suffer from hypoglycemia are illustrated in Figure 4. In this Figure, line "A" represents an ideal blood glucose level of 100 mg/100 ml. Line "B" represents a hypoglycemic's blood glucose level during a day when meals are eaten at around 8 o'clock, 12 o'clock and 6 o'clock. It will be seen that the blood glucose level both rises above and falls below the ideal range of 75-125 mg/100 ml. When it falls below the lower limit of 75 mg/100 ml, then the individual can suffer from hypoglycemic shock which can be very serious. In contrast, the line "C" represents the individual's blood glucose level when fed with the same meals as in "B" but in which the carbohydrate-containing parts are coated with a cation cross-linked polysaccharide in accordance with the present invention. It will again be seen that such a coating causes the blood glucose level to be smoothed out because of the slower digestion of the polysaccharide-containing components of the food in the individual's small intestine.

The uses and products provided by the present invention therefore have many advantages over previously available foods. Firstly, the cross-linked polysaccharide coating material such as alginate, alginic acid, pectin, pectinic acid, a pectinate or a pectate can easily be coated on the food by using standard cooking procedures such as boiling or simmering in water. Thus, both the use of fats as the coating or the need to use a non-aqueous organic solvent to apply the food coating are avoided.

The practical advantages of the uses and foods of the present invention are many fold. Thus, the invention provides a food-based strategy to control the release of glucose from carbohydrate-containing foods. The resulting foods have no marked difference in their palatability or taste from uncoated foods. The same foods can therefore be eaten by both individuals suffering from e.g. diabetes and those who do not. For people suffering from diabetes, the foods allow them improved control over their blood glucose levels. In healthy individuals, they also provide a feeling of prolonged satiety and so help to prevent snacking between meals. Further, because the coated foods take longer to digest in the small intestine, they give rise to prolonged release of glucose into the blood stream compared to normal foods. Such coated foods can therefore assist the performance of an individual during prolonged physical exercise during which glucose is constantly required by the muscles as an energy source.

The following provides a brief description of each of the Figures which are included in this specification:

Figure 1 is a hypothetical illustration showing the glycemic response of an individual to normal (uncoated) food,
Figure 2 is a hypothetical illustration showing the glycemic response of an individual to coated food in accordance with the present invention,
Figure 3 is a hypothetical illustration of the varying blood glucose levels of a healthy person and a diabetic person fed uncoated foods; and a diabetic person fed coated foods in accordance with the present invention,
Figure 4 is a hypothetical illustration showing the blood glucose levels of an individual suffering from hypoglycemia when fed uncoated and coated foods,
Figure 5 is a graph which illustrates how the percentage of glucose released from a coated food varies with the amount of coating applied around the food, and
Figure 6 is a graph which illustrates glucose release from a food coated with varying thicknesses of coating.

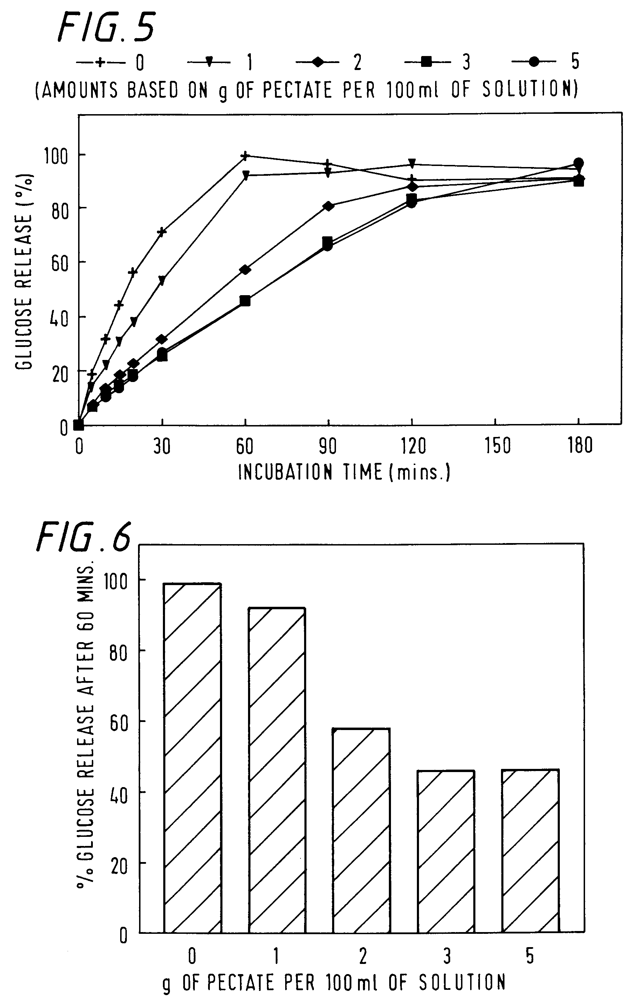

Figures 1-4 have already been referred to and discussed above. Figures 5 and 6 are discussed in detail in connection with Example 1 below.

The invention will now be described with reference to specific Examples which represent preferred embodiments of the present invention. These Examples should not however be considered to limit the scope of the appended claims.

Example 1 - Two-step coating of rice grains

(a) Coating with cross-linkable pectate

10 g of long grain white rice was washed and then added to an aqueous solution of 100 ml of 2% of the pectate LM1912CSZ (2 g per 100 ml of solution) obtainable from Hercules Inc. Pectate LM1912CSZ is a low methoxyl pectin with a degree of esterification of around 3% and a molecular weight of around 70,000-90,000 based upon its relative viscosity. The solution was boiled for around 16 minutes and the excess solution then poured away.

The resulting rice was then dried over-night at 90°C in a dry incubator. This step produces parboiled rice grains coated with a cross-linkable pectate coating. In fact, due to rice including some natural content of calcium cations, a relatively small amount of the pectate is already cross-linked at this intermediate stage. However, the majority of the pectate is not cross-linked and so the coating is quite elastic at this stage.

(b) Cross-linking of the pectate coating

25 ml of an aqueous solution containing 1% by weight of calcium chloride was heated to 100°C. 5 g of the intermediate dried rice product produced according to the above procedure (a) was added to the solution which was then boiled for 5 minutes. During this boiling the parboiled rice grains became fully cooked, and the cross-linkable pectate coating their surface became cross-linked due to the calcium cations present in the solution. After boiling the solution for 5 minutes, excess liquid was poured away to obtain the final coated rice product ready for eating. This rice product was found to include about 1.6% by weight of the cross-linked pectate coating.

Further rice products were obtained by following the above steps (a) and (b) except that the concentration of the pectate LM1912CSZ was varied in step (a) to be 1% by weight, 3% by weight and 5% by weight. A control experiment was also carried out in which no pectate was contained in the solution used in step (a). Accordingly, five different rice products were obtained in which the starting concentration of the pectate in step (a) was 0%, 1%, 2%, 3% and 5%.

(c) In vitro glucose-release characteristics of the rice products

Each of the five rice products previously obtained was subjected to an in vitro digestion assay to establish the rate at which the products release glucose. The in vitro assay method used was as follows.

The assay makes use of the following five reagents.

Enzyme Solution I.
Amyloglucosidase activity 13 AGU/ml Invertase activity 200 EU/ml Pancreatine, amylase activity 3800 BPU/ml

Enzyme Solution II.
Amyloglucosidase activity 50 AGU/ml

Glucose oxidase colorimetric kit.
Glucose GOD-PAP, Boehringer Mannheim, Cat. no. 166391.


Glucose standard solution.
Dissolve 50 mg of glucose in 50 ml distilled water (1 mg/ml). Concentrations of the standards 0-0.8 mg/ml 50 $\mu$l standard solution/sample + 1000 $\mu$l reagent mixture of the glucose oxidase colorimetric kit.


Sodium acetate buffer (0.1M).
Dissolve 13.6 g of sodium acetate trihydrate in 250 ml saturated benzoic acid solution, adjust the pH to 5.2, add 4 ml of 1M $CaC\ell_2$ and make to 1 $\ell$ with distilled water.

For each of the five rice products, approximately 5 g of the cooked rice was weighed exactly. The rice was then dried over-night in an oven at 90°C and weighed the next day to determine its dry matter. A further sample of each rice was masticated in a mincer with a plate having 0.9 cm diameter holes obtained from the Kitchen Basic range marketed by Boots, Nottingham, UK. Then, an exactly weighed amount of about 2.5 g of each of the five masticated rices was added to 20 ml of the sodium acetate buffer contained in five separate 50 ml Falcon tubes. Then, five glass balls were added. The tubes were pre-incubated in a shaking water bath at 37°C for five minutes. Then digestion was initiated with 5 ml of the Enzyme Solution I. 0.5 ml samples were then taken after 5, 10, 15, 20, 30, 60, 90, 120 and 180 minutes and pipetted into 1.0 ml of 96% ethanol. This was then centrifuged for 2 minutes at 3000 rpm. 50 $\mu$l of the resulting solution was then diluted with 1000 $\mu$l of distilled water. The glucose content was then measured using a glucose standard solution according to the following equation:

$$FG = [DF \times (V + A)]/Z$$

wherein FG is the free glucose expressed as mg/g dry

DF is the dilution factor (1.05/0.05 x 1.5/0.5)
V is the volume left in the incubation (after 5 minutes, this is 25 ml; after 10 minutes this is 24.5 ml etc.)
A is the amount (ml) of cooked rice in the incubation (e.g. 2.5g = 2.5 ml)
Z is the amount (g) of cooked rice in the incubation as measured dry.

After 180 minutes, each of the five samples contains 20.5 ml of solution and the cooked rice. The tubes were then vigorously shaken and placed in a boiling water-bath for 30 minutes. Then the tubes were cooled down to 0°C and 10 ml of 7M KOH was added. The tubes were then placed in a shaking water-bath with ice for 30 minutes.

In advance, Falcon tubes were prepared with 50 ml of 0.1 M acetic acid. Then 1.0 ml of the alkaline solution was pipetted into the acetic acid solution followed by 0.2 ml of the Enzyme Solution II. The tubes were shaken and placed in a water-bath at 60°C. After 30 minutes, the tubes were placed in a boiling water-bath for 10 minutes followed by cooling and centrifuging for 5 minutes at 1500g to remove the precipitate. Total glucose was then determined by pipetting 50 $\mu$l of the resulting solution into 1000 $\mu$l of the reagent mixture of the glucose oxidase colorimetric kit and the glucose measured against a standard curve. The total glucose is defined by the following equation:

$$TG = [DF \times \{(V_2 + A)/(V_1 + A)\} \times (V + A)]/Z$$

wherein TG is the total glucose (mg/g dry)

$V_1$ is 20.5 ml, $V_2$ is 30.5 ml, V is 25 ml
A is the amount (ml) of cooked rice in the incubation (e.g. 2.5 g = 2.5 ml)
Z is the amount (g) of cooked rice in the incubation as dry.

The percentage of glucose released is defined by the equation $(FG \times 100)/TG$.

The results of the various assays are illustrated in the graph forming Figure 5. It is apparent that the greater the pectate concentration used in step (a) during coating of the rice grains, then the slower the glucose release is with time in the *in vitro* assay. Thus, in the control where no pectate was used, all of the glucose was released after about 60 minutes. When a 1% solution of pectate was used, then the glucose-release was marginally slower, perhaps being complete after about 90 minutes. In contrast, when the initial pectate solution contained 2% by weight of pectate or more, then 60% or less of the glucose was released after 60 minutes. Further, glucose release continues steadily for around 120-150 minutes. Accordingly, it can be seen that the rate of glucose release, which corresponds roughly to the initial slope of the curves illustrated in Figure 5, decreases as the relative amount of pectate coating the rice grains increases. This effect is clearly illustrated in Figure 6 in which the percentage of glucose released after 60 minutes for each of the five rice samples is illustrated as a bar graph. The data for this graph was taken from the data illustrated in Figure 5 after an *in vitro* incubation time of 60 minutes.

The following Examples 2-11 demonstrate that the coating procedure provided as one aspect of the present invention results in a product which results in a slower release rate of glucose in an *in vitro* digestion system. Example 11 shows that a coating of calcium stearate as described at line 38, column 2 of US-A-5360614 is not resistant to physiological concentrations of bile acids.

Each of the Examples 2-11 measures glucose release according to the *in vitro* Englyst assay method. Two hours of *in vitro* incubation according to the Englyst assay method results in an extent of digestion which generally corresponds to a period of around 4-5 hours in the small intestine. Accordingly, it can be understood that the incubation conditions according to the Englyst assay method result in accelerated glucose release from a food as compared to digestion *in vivo*.

### Example 2

100g of washed long grain white rice was cooked in 227 ml of a 1% (wt/vol) sodium alginate (Sigma Chemicals) solution in distilled water for 10 minutes (all water absorbed). The rice was then dried over night in a dry incubator at 50°C. Subsequently 20 g of the dried rice was soaked in 100 ml of a 1% (wt/vol) $CaCl_2$ solution for 1 h at room temperature. After over night drying (55°C), 5 g of the rice was cooked for 3 minutes in a 25 ml 1% $CaCl_2$ solution. After pouring off the excess water, the cooked rice was subjected to digestion according to the *in vitro* assay method of Englyst discussed above. For control rice the same procedure was applied except sodium alginate was not present in the first cooking step.

| Glucose-release (% of total) | | |
|---|---|---|
| Time (minutes) | Control rice | Ca-alginate coated rice |
| 0 | 0 | 0 |
| 5 | 12 | 9 |
| 20 | 37 | 25 |
| 30 | 55 | 36 |
| 60 | 88 | 67 |
| 90 | 92 | 97 |
| 180 | 101 | 98 |

### Example 3

200 g of washed long grain white rice was cooked in 450 ml of a 2% (wt/vol) sodium alginate (Sigma Chemicals) solution in distilled water for 13 minutes (all water absorbed). The cooked rice was dried over night in a dry incubator at 70°C. 5 gram of the dried rice was cooked in 25 ml of a 1% (wt/vol) $CaCl_2$ solution for 3 minutes. After pouring off the excess water, the cooked rice was subjected to *in vitro* digestion according to the method of Englyst. For control rice the same procedure was applied except for the presence of sodium alginate in the first cooking step.

| Glucose-release (% of total) | | |
|---|---|---|
| Time (minutes) | Control rice | Ca-alginate coated rice |
| 0 | 0 | 0 |
| 5 | 8 | 4 |
| 20 | 25 | 12 |
| 30 | 37 | 17 |
| 60 | 68 | 30 |
| 90 | 88 | 46 |
| 120 | 94 | 60 |
| 180 | 90 | 89 |

Example 4

The same procedure as in Example 3 was followed except 20 g of the dried, cooked rice was soaked for 1 hour at room temperature in a 1% (wt/vol) $CaCl_2$ solution. After over-night drying (70°C), 5 g of the rice was cooked for 3 minutes in a 25 ml 1% $CaCl_2$ solution. The resulting cooked rice was analyzed using the same methodology as used in Example 3.

| Glucose-release (% of total) | | |
|---|---|---|
| Time (minutes) | Control rice | Ca-alginate coated rice |
| 0 | 0 | 0 |
| 5 | 16 | 5 |
| 20 | 43 | 14 |
| 30 | 60 | 20 |
| 60 | 84 | 40 |
| 90 | 92 | 58 |
| 120 | 98 | 75 |
| 180 | 91 | 107 |

Example 5

The procedure of Example 4 was followed except that the rice was cooked for 5 minutes in 1% $CaCl_2$ solution instead of 3 minutes as in Example 4.

| Glucose-release (% of total) | | |
|---|---|---|
| Time (minutes) | Control rice | Ca-alginate coated rice |
| 0 | 0 | 0 |
| 5 | 16 | 7 |
| 20 | 52 | 19 |
| 30 | 68 | 28 |
| 60 | 88 | 56 |
| 90 | 98 | 78 |
| 120 | 102 | 82 |
| 180 | 98 | 89 |

### Example 6

75 g of washed long grain rice was cooked in 168 ml of a polymer solution in distilled water for 20 minutes (all water absorbed). The cooked rice was dried over-night in a dry incubator at 70°C. 5 gram of the dried rice was cooked in 25 ml of a 1% (wt/vol) $CaCl_2$ solution for 5 minutes. After pouring off the excess water, the cooked rice was subjected to *in vitro* digestion according to the Englyst method. For control rice the same procedure was applied except that no polymer was used.

The following polymers were used. Pectin X-4912 (CPF, Denmark) 1% wt/vol, sodium alginate (Sigma Chemicals) 2% wt/vol, sodium alginate (Pronova Biopolymer, Inc.) 1% wt/vol, and pectin LM1912CSZ (Hercules Inc.) 1% wt/vol.

| Glucose-release (% of total) | | | | | |
|---|---|---|---|---|---|
| Time (minutes) | Control | X-4912 | LM1912CSZ | alginate (Sigma) | alginate (Pronova) |
| 0 | 0 | 0 | 0 | 0 | 0 |
| 5 | 19 | 16 | 9 | 7 | 10 |
| 20 | 47 | 42 | 22 | 18 | 25 |
| 30 | 60 | 58 | 31 | 26 | 33 |
| 60 | 82 | 84 | 59 | 51 | 63 |
| 90 | 90 | 89 | 74 | 70 | 82 |
| 120 | 92 | 89 | 88 | 84 | 85 |
| 180 | 89 | 92 | 84 | 91 | 92 |

### Example 7

10 g of washed long grain white rice was cooked in 100 ml of a pectin LM1912CSZ solution (2% wt/vol) in distilled water for 16 minutes. After pouring off the excess water, the cooked rice was dried over night in a dry incubator at 90°C. 3 gram of the dried rice was cooked in 15 ml of a 1% (wt/vol) $CaCl_2$ solution or in distilled water for 5 minutes. After pouring off the excess water, the cooked rice was subjected to *in vitro* digestion according to the Englyst method, or according to this method without the presence of marbles. For control rice the same procedure was applied except that no pectin was used.

| Glucose-release (% of total) | | |
|---|---|---|
| Time (minutes) | 20 | 120 |
| -marbles/-Ca | | |
| Control | 56 | 108 |
| Pectin | 26 | 77 |
| +marbles/-Ca | | |
| Control | 81 | 118 |
| Pectin | 31 | 106 |
| -marbles/+Ca | | |
| Control | 55 | 107 |
| Pectin | 32 | 87 |
| +marbles/+Ca | | |
| Control | 78 | 116 |
| Pectin | 38 | 117 |

Example 8

10 g of washed long grain rice was cooked in 25 ml of the pectin LM1912CSZ solution (2% wt/vol) in distilled water for 15 minutes (all water absorbed). Following this, the same procedure was applied as used in Example 7.

| Glucose-release (% of total) | | |
|---|---|---|
| Time (minutes) | 20 | 120 |
| -marbles/-Ca | | |
| Control | 41 | 107 |
| Pectin | 21 | 82 |
| +marbles/-Ca | | |
| Control | 63 | 116 |
| Pectin | 26 | 103 |
| -marbles/+Ca | | |
| Control | 41 | 100 |
| Pectin | 22 | 78 |
| +marbles/+Ca | | |
| Control | 53 | 109 |
| Pectin | 25 | 103 |

Example 9

10 g of washed long grain white rice was cooked in 100 ml of a pectin LM1912CSZ or alginate (Pronova) solution (2% wt/vol) in distilled water for 16 minutes. After pouring off the excess water, the cooked rice was dried over- night in a dry incubator at 90°C. 3 gram of the dried rice was cooked in 15 ml of a 1% (wt/vol) $CaCl_2$ solution or in distilled water for 5 minutes. After pouring off the excess water, the cooked rice was subjected to *in vitro* digestion according the Englyst method and according to this method without the presence of marbles. For control rice the same procedure was applied except for the absence of pectin or alginate. The whole experiment was repeated three times, except for the alginate.

| Glucose-release as % of total (± standard deviation) | | | |
|---|---|---|---|
| Time (minutes) | Control (- marbles) | LM1912CSZ (- marbles) | Alginate (- marbles) |
| 0 | 0 | 0 | 0 |
| 5 | 18(1) | 11(1) | 10 |
| 20 | 42(2) | 27(1) | 23 |
| 30 | 55(5) | 35(1) | 32 |
| 60 | 77(2) | 57(3) | 48 |
| 90 | 91(2) | 70(1) | 62 |
| 120 | 94(4) | 81(3) | 65 |
| 180 | 98(5) | 90(1) | 92 |

| Glucose-release as % of total (± standard deviation) | | | |
|---|---|---|---|
| Time (minutes) | Control (+ marbles) | LM1912CSZ (+ marbles) | Alginate (+ marbles) |
| 0 | 0 | 0 | 0 |
| 5 | 20(1) | 12(1) | 12 |
| 20 | 52(1) | 29(1) | 37 |
| 30 | 69(2) | 38(3) | 47 |
| 60 | 92(1) | 66(9) | 84 |
| 90 | 97(1) | 84(2) | 106 |
| 120 | 98(2) | 89(9) | 95 |
| 180 | 96(1) | 89(8) | 101 |

Example 10

Rice was cooked according to Example 7 with the cooking step in distilled water. Half of the rice was masticated using a mincer with a plate of 0.9 cm diameter holes (Kitchen Basics range, Boots, Nottingham, Great Britain). *In vitro* digestion was carried out according to the Englyst method. Coating materials used were pectin LM1912CSZ and alginate (Pronova).

| Glucose-release as % of total without mastication | | | |
|---|---|---|---|
| Time (minutes) | Control | LM1912CSZ | Alginate |
| 0 | 0 | 0 | 0 |
| 5 | 16 | 11 | 10 |
| 20 | 40 | 30 | 26 |
| 30 | 52 | 36 | 35 |
| 60 | 76 | 59 | 55 |
| 90 | 88 | 69 | 69 |
| 120 | 93 | 81 | 76 |
| 180 | 93 | 87 | 91 |

| Glucose-release as % of total with mastication | | | |
|---|---|---|---|
| Time (minutes) | Control | LM1912CSZ | Alginate |
| 0 | 0 | 0 | 0 |
| 5 | 23 | 14 | 14 |
| 20 | 52 | 33 | 35 |
| 30 | 63 | 43 | 45 |
| 60 | 83 | 63 | 70 |
| 90 | 87 | 70 | 80 |
| 120 | 90 | 82 | 83 |
| 180 | 95 | 88 | 94 |

Example 11

Corn starch was coated with either Ca-pectate or with Ca-stearate and digested *in vitro* according to the Englyst method with or without 40 mM of bile acid (cholic acid). Coating with Ca-stearate was done as follows. 5 g corn starch was suspended in methanol. A solution containing 0.75 g CaCl$_2$ 2H$_2$O in methanol was added. After stirring for a while a solution containing 1.55 g sodium stearate in methanol was added. After mixing the suspension was filtered and dried.

| Glucose-release as % of total without bile acids | | | |
|---|---|---|---|
| Time (minutes) | Control | Ca-pectate | Ca-stearate |
| 0 | 0 | 0 | 0 |
| 5 | 63 | 27 | 13 |
| 20 | 109 | 52 | 14 |
| 30 | 110 | 61 | 22 |
| 60 | 112 | 86 | 45 |
| 90 | 108 | 92 | 68 |
| 120 | 107 | 102 | 80 |
| 180 | 106 | 96 | 85 |

| Glucose-release as % of total with bile acids | | | |
|---|---|---|---|
| Time (minutes) | Control | Ca-pectate | Ca-stearate |
| 0 | 0 | 0 | 0 |
| 5 | 43 | 25 | 21 |
| 20 | 67 | 48 | 43 |
| 30 | 80 | 66 | 60 |
| 60 | 108 | 83 | 97 |
| 90 | 102 | 97 | 97 |
| 120 | 99 | 95 | 108 |
| 180 | 99 | 94 | 101 |

**Claims**

1. The use of a cation cross-linked polysaccharide coating for reducing the glycemic response of a carboyhdrate-containing food.

2. The use of a cation cross-linked polysaccharide in the coating of a carbohydrate-containing food for reducing the glycemic response of the food.

3. The use according to Claim 1 or Claim 2, wherein the polysaccharide is alginate, alginic acid, pectin, pectinic acid, a pectinate, a pectate, polygalacturonic acid, carrageenan, cross-linkable cellulose or a derivative thereof, xanthan gum, agar, cross-linkable starch or cross-linkable guar gum.

4. The use according to any preceding Claim, wherein the polysaccharide is cross-linked with calcium cations or magnesium cations.

5. The use according to any preceding Claim, wherein the cross-linked polysaccharide coating surrounds the food to fully encase it.

6. The use according to any preceding claim, wherein the coated food comprises 0.01 - 5% by weight (on a dry weight basis) of the cation cross-linked polysaccharide.

7. The use according to Claim 6, wherein the coated food comprises 1 - 3% by weight (on a dry weight basis) of the cation cross-linked polysaccharide.

8. The use according to any preceding Claim, wherein the carbohydrate-containing food includes peptidoglycans, polysaccharides, oligosaccharides, disaccharides, monosaccharides or sugar alcohols, or any combination thereof.

9. The use according to any preceding Claim, wherein the carbohydrate is one or more of starch, dextrin, sucrose, mannose, maltose, glucose, fructose, lactitol, xylitol, lactose, sorbitol and mannitol.

10. The use according to any preceding Claim, wherein the food is a rice grain, pasta shape, a vegetable or part thereof, a flour granule or granules, a starch granule or granules or a piece of breakfast cereal.

11. The use according to any preceding Claim, wherein the food has a longest dimension of less than 3 cm.

12. The use according to any of Claims 1-10, wherein the food has an elongate shape with a cross sectional area of no more than 1cm$^2$.

13. The use according to any preceding Claim, wherein the coated food is a food ingredient used in combination with one or more other ingredients to prepare a food product.

14. The use according to Claim 13, wherein the food product comprises at least 10% by weight of the coated food.

15. The use according to any preceding claim, wherein the glycemic response of the coated food is reduced by at least 20% compared to an uncoated food 30 minutes after digestion based on the *in vitro* Englyst assay method.

16. The use according to any preceding claim, wherein the glycemic response of the coated food is reduced by at least 20% compared to an uncoated food 60 minutes after digestion based on the *in vitro* Englyst method.

17. A coated food comprising a core of a carbohydrate-containing hydrated food and a coating layer comprising a cation cross-linked polysaccharide which is insoluble in boiling water.

18. A coated food according to Claim 17, wherein the hydrated food core is a cooked grain of rice or pasta shape.

19. A food for cooking comprising:

   (i) a core of a carbohydrate-containing food coated with a cross-linkable polysaccharide coating; and
   (ii) a source of cross-linking cations for cross-linking the polysaccharide,

the components (i) and (ii) being separate or in physical admixture.

20. A food according to Claim 19, comprising 0.01 - 20% by weight of the cross-linking cations based on the amount of the cross-linkable polysaccharide.

21. A food according to Claim 19 or Claim 20, wherein the core is a grain of parboiled rice.

22. A method for preparing a cation cross-linked polysaccharide coated carbohydrate-containing food comprising the steps of:

(i) coating a cross-linkable polysaccharide on a hydratable carbohydrate-containing food core; and
(ii) hydrating the food core by cooking the coated food core in an aqueous solution of cross-linking cations which simultaneously cross-link the coating.

23. A method according to Claim 22, wherein the coating step comprises spraying or dipping the food core with or in an aqueous solution of the cross-linkable polysaccharide at a temperature of 40-100°C.

24. A method according to Claim 23, wherein the cross-linkable polysaccharide is present at a concentration of 0.01 - 10% by weight of the aqueous solution.

25. A method according to any of Claims 22-24, further comprising the step of drying the coated hydratable core prior to the hydrating step (ii).

26. The use of a cation cross-linked polysaccharide coated carbohydrate-containing alimentary material as a food for an individual suffering from diabetes, hypoglycaemia or glycogen storage disease.

27. The use of a cation cross-linked polysaccharide coated carbohydrate-containing food as an appetite suppressant or to assist in performing sustained physical activity.

EP 0 749 697 A1

FIG. 1

FIG. 2

18

## FIG. 3

## FIG. 4

## FIG.5

—+— 0    —▼— 1    —◆— 2    —■— 3    —●— 5

(AMOUNTS BASED ON g OF PECTATE PER 100 ml OF SOLUTION)

## FIG.6

**European Patent Office**

# EUROPEAN SEARCH REPORT

Application Number

EP 95 10 9753

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | US-A-5 360 614 (J. GARY FOX ET AL.)<br><br>* abstract; claims 1-3; examples 4,6,11 *<br>* column 1, line 1 - column 2, line 21 *<br>--- | 1,3-5,8,<br>9,11,12,<br>17,26,27 | A23P1/08<br>A23L1/09<br>A23L1/29<br>A23L1/10 |
| Y | FR-A-1 444 303 (A.E. STALEY MANUFACTURING COMPANY)<br><br>* claims 1-7 * | 1,3-5,8,<br>9,11,12,<br>17,26,27 | |
| A | --- | 22-24 | |
| Y | EP-A-0 487 340 (HERCULES INCORPORATED)<br><br>* page 2, line 49 - line 55 *<br>* page 4, line 15 - line 20 *<br>--- | 1,3-5,8,<br>9,11,12,<br>17,26,27 | |
| D,A | US-A-2 611 708 (HARRY S. OWENS ET AL.)<br>* examples 1,7,8 *<br>--- | 22 | |
| A | DATABASE WPI<br>Week 9005<br>Derwent Publications Ltd., London, GB;<br>AN 90-034381<br>& JP-A-01 313 421 (ALIMENT KOGYO KK) , 18 December 1989<br>* abstract *<br>--- | | **TECHNICAL FIELDS SEARCHED** (Int.Cl.6)<br><br>A23P<br>A23L |
| D,A | US-A-2 517 595 (HARRY S. OWENS ET AL.)<br>----- | | |

The present search report has been drawn up for all claims

| Place of search | Date of completion of the search | Examiner |
|---|---|---|
| BERLIN | 23 November 1995 | Alvarez Alvarez, C |

EPO FORM 1503 03.82 (P04C01)